# EUROPÄISCHE PATENTSCHRIFT

(11) **EP 1 625 837 B1**
(45) Veröffentlichungstag und Bekanntmachung des Hinweises auf die Patenterteilung: **09.05.2007**
(21) Anmeldenummer: 04019159.5
(22) Anmeldetag: 12.08.2004
(51) Int. Cl.: A61F 9/013

(54) **Mikrokeratom sowie chirurgische Klinge hierfür**
Microkeratome as well as surgical blade for it
Microkératome et lame de coupe chirurgicale pour celui-ci

(43) Veröffentlichungstag der Anmeldung: 15.02.2006
(73) Patentinhaber: Gebauer GmbH & Co. KG, 75242 Neuhausen (DE)
(72) Erfinder: Gebauer, Detlev Paul, 75233 Tiefenbronn (DE)
(74) Vertreter: Hofer, Dorothea

(56) Entgegenhaltungen:
- WO-A-03/034958
- WO-A-20/04052254
- DE-A- 10 119 477
- US-A- 5 980 543
- US-A1- 2003 018 348

## Beschreibung

Die vorliegende Erfindung bezieht sich auf ein Mikrokeratom sowie auf eine chirurgische Klinge für ein Mikrokeratom. Insbesondere bezieht sich die Erfindung auf ein Mikrokeratom und auf eine dazugehörige Klinge, die zwar nach der LASIK-Methode eingesetzt wird, jedoch die Vorteile der LASEK-Methode aufweist, ohne deren Nachteile zu übernehmen. Im Folgenden werden kurz die LASIK- und die LASEK-Methode erläutert.

Die sog. LASIK-Methode (LASer In-situ-Keratomileusis) ist ein chirurgisches Augenoperationsverfahren zur Korrektur einer Fehlsichtigkeit des Auges mit Hilfe eines Excimer-Lasers, das derzeit am häufigsten angewandt wird. Bei der LASIK-Methode wird von der Augenhornaut eine Hornhautlamelle mit einer Dicke von ca. 150-160 µm abgehobelt. Dies geschieht mit Hilfe eines Mikrokeratoms (Spezialhobel). Allerdings wird nicht die gesamte Hornhautlamelle abgehobelt, sondern es bleibt ein kleines Reststück mit der übrigen Hornhaut verbunden. Das nicht abgehobelte Reststück dient als eine Art Scharnier (Hinge), an dem die abgehobelte Hornhautlamelle weggeklappt wird. Anschließend wird das freiliegende Gewebe mit Hilfe eines computergesteuerten Excimer-Lasers in die gewünschte Form gebracht, so daß die Fehlsichtigkeit korrigiert wird. Danach wird die Hornhautlamelle wieder zurückgeklappt und verschließt das bearbeitete Gewebe. Dadurch wird ein Vernähen unnötig, eine schnelle Heilung unterstützt und eine Narbenbildung verhindert.

Alternativ dazu hat sich mittlerweile die sog. LASEK-Methode (LASer Epitheiiale-Keratomileusis) etabliert, die in der jüngsten Vergangenheit immer häufiger angewandt wird.

Bei der LASEK-Methode wird nur die oberste ca. 55 µm dicke Schicht der Augenhornhaut, das sog. Epithelium, von der Augenhornhaut entfernt. Das darunterliegende Stromagewebe bleibt unversehrt. Dies geschieht mit Hilfe einer speziellen Vorrichtung, dem sog. Mikrotrepan, der ein Rundmesser mit einer Schneidklinge in einem Bereich von ca. 270° aufweist. Der Mikrotrepan wird auf das Auge aufgesetzt und dringt ca. 55 µm tief in das Epithelium ein und wird anschließend mehrmals um ca. 10° hin und her gedreht. Dadurch wird das Epithelium in einem Bereich von ca. 280° kreisrund eingeschnitten. Der nicht geschnittene Bereich von ca. 80° bleibt ähnlich wie bei der LASIK-Methode ebenfalls als eine Art "Scharnier" (hinge) übrig, an dem das geschnittene Epithelium hängt. Das Rundmesser wird für den Schnitt in einem Zylinder geführt, der auf das Auge aufgesetzt wird. Nach dem Schnitt wird eine verdünnte Alkohollösung in einen auf dem Auge aufgesetzten Zylinder geträufelt, so das die Alkohollösung mit dem geschnittenen Epithelium in Kontakt gelangt. Die Alkohollösung dient dazu, das Gewebe aufzuweichen. Nach ca. 30 Sekunden wird die Alkohollösung abgesaugt und das eingeschnittene Epithelium wird mit Hilfe eines Spatels auf die Seite des Scharniers geschoben. Nach diesen vorbereitenden Maßnahmen wird die eigentliche Laserbehandlung auf dem freiliegenden Gewebe wie bei der vorbeschriebenen LASIK-Methode vorgenommen.

### Nachteile des bisherigen LASEK-Verfahrens

Um das eingeschnittene Epithelium aufzuweichen und beseite schieben zu können, ist der Einsatz von Alkohol notwendig. Der Einsatz von Alkohol ist allerdings aus folgenden Gründen problematisch: Erstens darf der Alkohol während der Behandlung keinesfalls auf das Bindegewebe neben dem eingeschnittenen Epithelium gelangen, da dies beim Patienten sehr große Schmerzen verursachen würde. Zweitens handelt es sich bei Alkohol um ein Nervengift, das grundsätzlich als schädlich erachtet wird.

Bislang ist zwar nicht eindeutig klar, wie tief der Alkohol in das Gewebe eindringt und welche Schäden dadurch hervorgerufen werden; als sicher gilt jedoch, daß Alkohol eine schädigende Wirkung hat. Drittens besteht ein nicht unerhebliches Risiko, daß während einer Operation die korrekte Dauer der Einwirkzeit des Alkohols auf dem Gewebe von 30 Sekunden nicht eingehalten wird.

Ein weiterer Nachteil ist, daß das beiseite Schieben des kreisrund eingeschnittenen Epitheliums vom Operateur eine große Geschicklichkeit erfordert und das Risiko, daß das Epithelium bei der Operation beschädigt oder unbrauchbar wird, nicht unerheblich ist.

Aus der US 2003/0018348 A1 ist eine Vorrichtung zum Abschaben des Epitheliums bekannt, die einen großen Rundungsradius zwischen 2 und 13 µm besitzt, mit der die Entfernung des Epitheliums ohne Verwendung einer Alkohollösung durchgeführt wird.

Aus der WO 97/20529 A1 ist eine Schneidvorrichtung für die Augenbehandlung bekannt, die eine Klinge mit einem Schneidenwinkel zwischen 0 und 30° besitzen kann, vorzugsweise 20° und noch weiter vorzugsweise 9°.

Darüber hinaus ist aus der WO 93/06783 A1 eine Schneidvorrichtung für die Augenbehandlung bekannt, bei der ein Schneidenwinkel in einem direkt an der Schneidenspitze liegenden Bereich 11° und in zwei weiteren sich daran anschließenden Bereichen 8° bzw. 6° beträgt.

Aus der WO2004/052254 ist ein Messer für ein Mikrokeratom entsprechend dem Oberbegriff des Anspruchs 1 bekannt.

Es ist Aufgabe der vorliegenden Erfindung, die oben beschriebenen Nachteile zu vermeiden, d.h. eine Operation nach der LASEK-Methode zu ermöglichen, die keine große Geschicklichkeit erfordert und eine saubere Trennung des Epitheliums von der Bowman-Membran ohne Alkohol ermöglicht.

Diese Aufgabe wird mit einem Messer nach Anspruch 1, einem Mikrokeratom nach Anspruch 5 und einer Verwendung eines Mikrokeratoms nach Anspruch 6 gelöst.

Vorteilhafte Weiterbildungen sind Gegenstand der Unteransprüche.

Bei der modifizierten LASEK-Methode wird das Epithelium nicht mit Hilfe des Mikrotrepans kreisrund eingeschnitten und mit einem Spatel beiseite geschoben. Vielmehr kann mit dem erfindungsgemäßen Mikrokeratom und der darin verwendeten definiert stumpfen Klinge die LASEK-Methode wie die LASIK-Methode durchgeführt werden. Das heißt, daß das Epithelium nicht mit Alkohol aufgeweicht wird, sondern mit Hilfe des Mikrokeratoms glatt abgehobelt wird, wobei ebenfalls ein Scharnier (hinge) belassen wird, das zur Rejustierung der abgehobelten Hornhautlamelle dient. Im Vergleich zur Schnittiefe von ca. 150-160 µm bei der LASIK-Methode erfolgt hier der Schnitt wie bei der LASEK-Methode in einer wesentlich geringeren Tiefe von nur ca. 55 µm. Dadurch bleibt die sog. Bowman-Membran unter dem Epithelium sowie das darunter liegende Stromagewebe unversehrt.

Erst mit der erfindungsgemäß definierten Klingengeometrie ist es möglich, das Epithelium sauber zu durchdringen und bis zur Bowman-Membran vorzustoßen, ohne diese zu verletzen. Eine zu scharfe Klinge würde auch die Bowman-Membran durchdringen und im Stromagewebe schneiden, was es jedoch zu vermeiden gilt. Eine zu stumpfe Klinge wäre nicht in der Lage, das Epithelium sauber von der Bowman-Membran zu lösen, so daß unter Umständen Zellen des Epitheliums auf der Bowman-Membran verbleiben könnten, was es ebenfalls zu vermeiden gilt.

Besonders vorteilhaft ist es, wenn der Übergang von dem Klingenradius in die Freifläche vorzugsweise völlig stufen- oder knickfrei, gestaltet ist. Dabei berührt die Freifläche bzw. eine gedachte Verlängerung der Freifläche der Klinge tangential die Rundung des Klingenradius. Idealerweise beträgt zudem die Klingenbreite mindestens 8mm, vorzugsweise zwischen 8-12mm. Die Härte des Klingenmaterials ist größer als die Härte von Blei, d.h. größer als 1,5 Härtegrad nach Mohs.

Die definiert stumpfe Messerklinge kann kostengünstig aus einem billigen Material wie beispielsweise Edelstahl gefertigt werden. Da Edelstahl mit herkömmlichen Schleifmethoden bearbeitet werden kann, ist der Schleifvorgang einfach und kostengünstig. Dadurch kann die Messerklinge als Einmalklinge verwendet werden, so daß die Messerklinge nach ihrem Einsatz weggeworfen wird. Vorteilhafter Weise entfällt dadurch die aufwendige Reinigung der Messerklinge und die Gefahr der Übertragung von Keimen etc. wird durch die Einmal-Verwendung ausgeschaltet. Insbesondere die Übertragung von Eiweißstoffen, den sog. Prionen, die für die Übertragung der Creutzfeld-Jacob Krankheit verantwortlich gemacht werden, kann ausgeschaltet werden. Dies ist besonders deshalb erwähnenswert, da die Prionen selbst bei der herkömmlichen Dampfsterilisation im Autoklaven überleben können.

Indem die LASEK-Methode mit Hilfe der erfindungsgemäßen Klinge nach der LASIK-Methode durchgeführt werden kann, kann das bisher für die LASIK-Methode angewandte Mikrokeratom dafür verwendet werden. Das heißt, das Mikrokeratom kann sowohl für die bisherige LASIK-Methode als auch für die LASEK-Methode eingesetzt werden. Es müssen lediglich die Messer ausgetauscht werden. Durch diese Doppelnutzung können erhebliche Kosten eingespart werden.

Außerdem ist die Operation mit Hilfe des Mikrokeratoms erheblich einfacher, da der Schnitt weitgehend automatisch erfolgt, so daß keine große Geschicklichkeit und Erfahrung erforderlich ist.

Nachfolgend wird die Erfindung anhand einer derzeit bevorzugten Ausführungsform unter Bezugnahme auf die beigefügten Figuren näher erläutert.
Fig. 1 ist eine perspektivische Ansicht eines Mikrokeratoms.
Fig. 2 ist eine Seitenansicht des Mikrokeratoms gemäß Fig. 1.
Fig. 3 ist eine Draufsicht auf das Mikrokeratom gemäß Fig. 1.
Fig. 4 ist eine vergrößerte Darstellung eines Kreisausschnitts aus Fig. 2.
Fig. 5 ist eine vergrößerte Teilansicht der chirurgischen Klinge und seiner Schneidgeometrie mit angedeuteter Kornea und Applanationsfläche.
Fig. 6 ist eine vergrößerte Teilansicht der chirurgischen Klinge.
Fig. 7 ist eine weitere vergrößerte Teilansicht der chirurgischen Klinge.

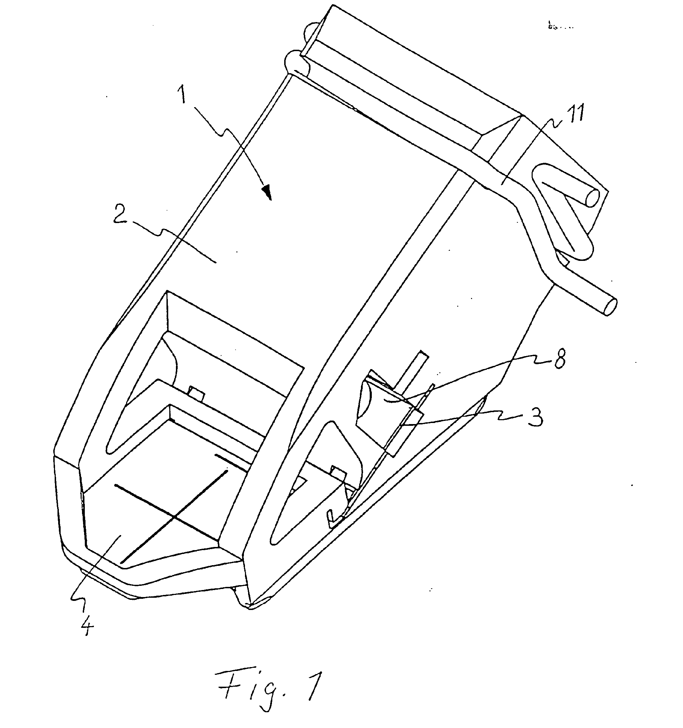

Fig. 1 zeigt ein Mikrokeratom 1, wie es üblicherweise auch für die bisherige LASIK-Methode angewandt wird. Da ein Mikrokeratom an sich bekannt ist, wird im Folgenden nur auf die wesentlichen Merkmale eingegangen, die in Verbindung mit der vorliegenden Erfindung von Bedeutung sind. Im übrigen wird hinsichtlich des Aufbaus eines Mikrokeratoms beispielsweise auf die EP-A-0 873 735 A1 verwiesen. Das Mikrokeratom besteht aus einem Schneidkopf 2, der eine chirurgische Klinge bzw. ein Messer 3 enthält. Am vorderen Ende des Schneidkopfs 2 befindet sich eine Applanationsfläche 4, die eine durchsichtige Scheibe aufweist, auf der ein Fadenkreuz abgebildet ist. Die Applanationsfläche 4 dient zur Auflage auf die zu schneidende Augenhornhaut (Kornea 14 in Fig. 5), wobei der Schneidkopf 2 auf einem (nicht dargestellten) Saugring angebracht wird, der auf dem Auge fixiert wird. Mit der Applanationsfläche 4 kann ein bestimmter Druck auf die Augenhornhaut aufgebracht werden, der den Schnitt mit dem Messer 3 günstig beeinflußt.

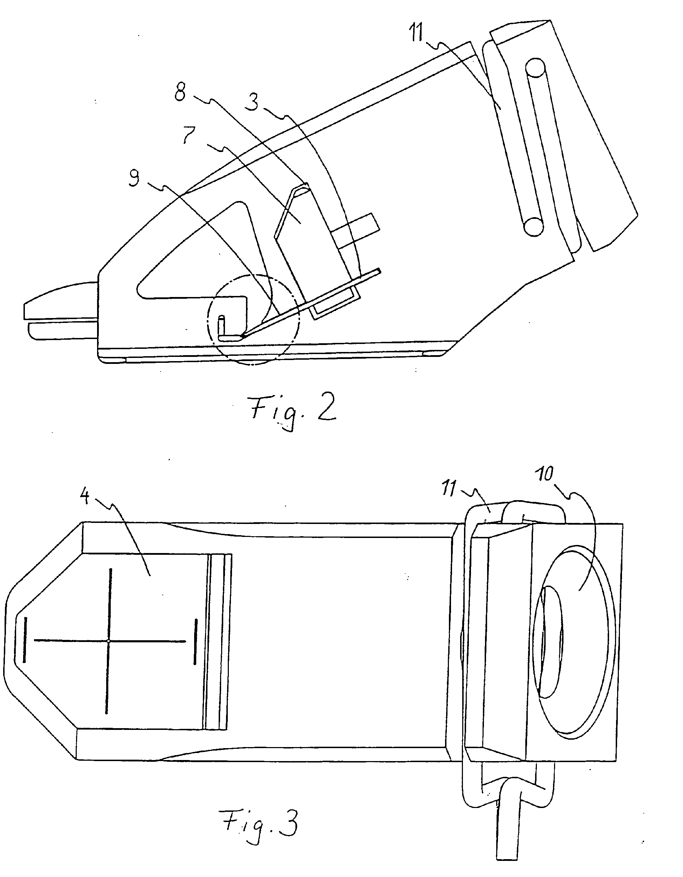

Gemäß Fig. 2 befindet sich seitlich in dem Schneidkopf 2 eine Aufnahmeöffnung 8, in die ein Messerhalter 7 eingeschoben wird. Der Messerhalter 7 enthält ein Messer 3. Im Schneidkopf 2 verläuft von der Aufnahmeöffnung 8 aus in Richtung zur Applanationsfläche 4 hin ein Spalt 9, der zur Aufnahme des Messers 3 dient, das im Messerhalter 7 gehalten wird.

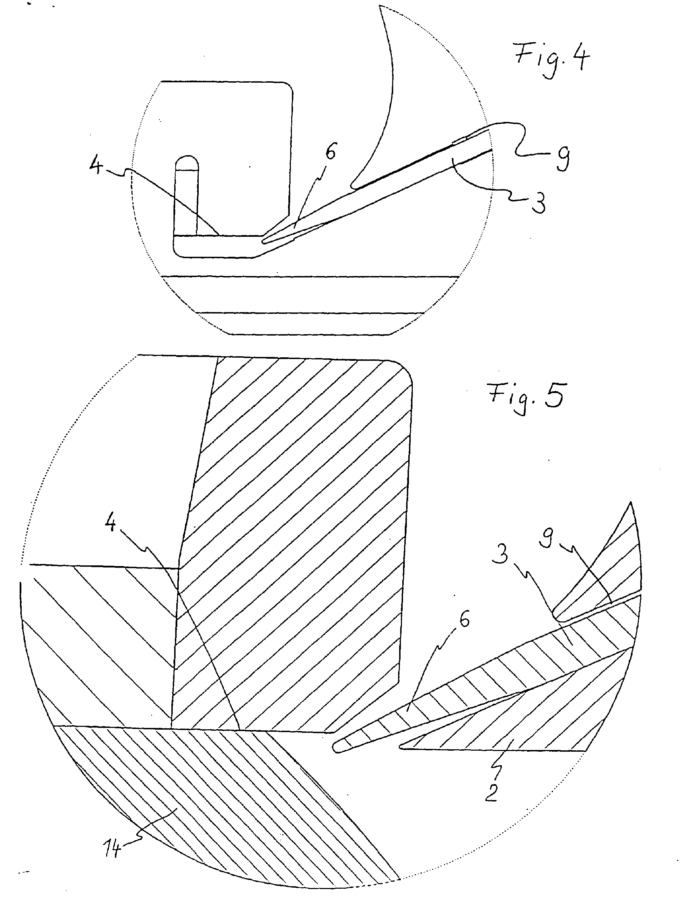

Wie in Fig. 3 gezeigt ist, befindet sich gegenüber der Applanationsfläche 4 in Längsrichtung des Schneidkopfs 2 gesehen, eine Kopplungsöffnung 10, in die ein Motorantrieb angeschlossen wird, mit dem das Messer 3 angetrieben wird, da das Messer im Schneidbetrieb wie ein Scherkopfmesser eines Rasierapparates parallel zur Klinge, d.h. in Fig. 1 von vorne aus gesehen von links nach rechts und wieder zurück, quer zur Schnittrichtung hin und her bewegt wird. Am Außenumfang des Schneidkopfs 2 ist im Bereich der Kopplungsöffnung 10 eine Verriegelung 11 vorgesehen, die dazu dient, den Motorantrieb in der Kopplungsöffnung zu fixieren. Die Oszillationsfrequenz, mit der die besten Ergebnisse erzielt werden können, liegt in einem Bereich von 3000 bis 10.000 U/min. Außerdem erfolgt die Bewegung des Messers 3 in der Schnittrichtung in dem Mikrokeratom automatisch, wobei die optimale Vorschubgeschwindigkeit zwischen 0,5 und 1,55 mm/sec. beträgt.

In Fig. 4 ist ein Kreisausschnitt aus Fig. 2 vergrößert dargestellt. Das Messer 3 erstreckt sich durch den Spalt 9 des Schneidkopfs 2 in Richtung zur Applanationsfläche 4. Dabei ragt die Klinge 6 des Messers 3 wie bei einem Hobel bis etwas unterhalb der Applanationsfläche 4, so daß ein Schnitt in einer Tiefe von ca. 55 µm durchgeführt werden kann.

Gemäß Fig. 5 drückt die Applanationsfläche 4 die Kornea 14 des zu behandelnden Auges flach, bevor die Klinge 6 des Messers 3 in das Epithelium eindringt.

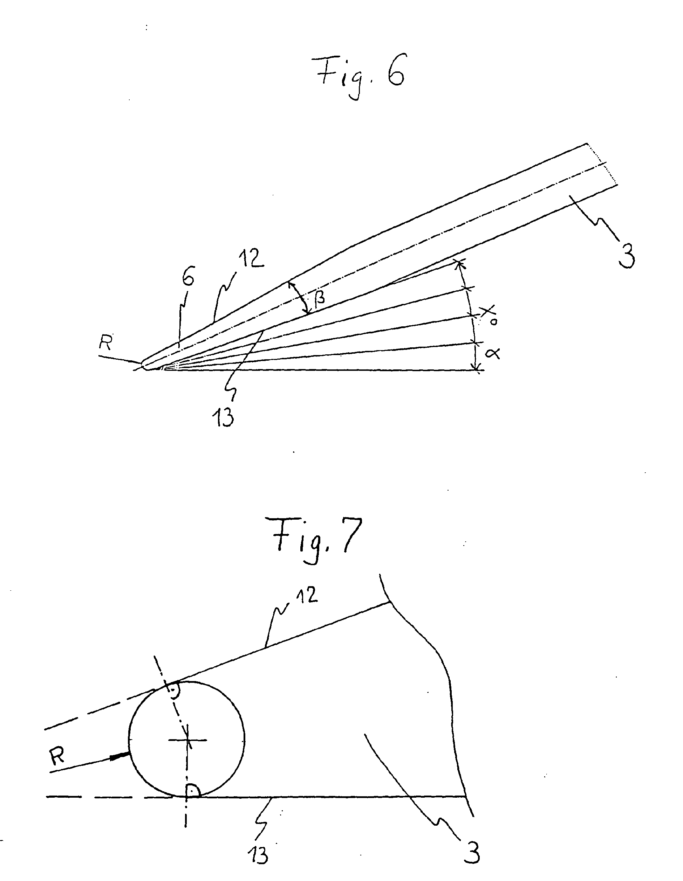

Im Folgenden wird die Geometrie der Klinge 6 unter Bezugnahme auf Fig. 6 näher erläutert. Die Klinge 6 weist eine obere Schneidfläche 12 und eine untere Schneidfläche 13 auf. Der Freiwinkel α ist der Winkel zwischen der unteren Schneidfläche 12 und einer imaginären horizontalen Linie, die der Applanationsfläche 4 entspricht. In der vorliegenden Ausführungsform beträgt der Freiwinkel ca. 4°. Der Freiwinkel kann jedoch in einem Bereich von 0 bis 4° variieren. Der Schneidenwinkel β ist der Winkel zwischen der oberen und der unteren Schneidfläche 12, 13. Er beträgt in der vorliegenden Ausführungsform ca. 30°. Der Freiwinkel kann jedoch in einem Bereich von 28 bis 35° variieren. Der Klingenradius R ist der Radius, mit dem die Klingenspitze gerundet ist. Er beträgt in der vorliegenden Ausführungsform ca. 450nm. Der Klingenradius kann jedoch in einem Bereich von 150-800 nm variieren.

In Fig. 7 ist gezeigt, daß die beiden Freiflächen der oberen und unteren Schneidflächen 12 und 13 tangential in den Klingenradius R übergehen. Der Übergang von dem Kleingenradius in die Freifläche ist vorzugsweise völlig stufen- oder knickfrei gestaltet. Zur Verdeutlichung der tangentialen Anlage der Freiflächen an dem Klingenradius ist der Klingenradius als imaginärer Vollkreise abgebildet und die Freiflächen sind als imaginär verlängerte Linien, die über den Klingenradius hinausgehen dargestellt. Dabei ist deutlich zu sehen, daß die Freifläche bzw. die imaginäre Verlängerung der Freifläche der Klinge die Rundung des Klingenradius tangential berührt.

Wird der Schnitt durchgeführt, dringt die Klinge 6 in das Epithelium bis zur Bowman-Membran in einer Tiefe von ca. 55 µm ein. Da die Bowman-Zellen etwas stabiler als das Epithelium sind, dringt die Klinge 6 aufgrund ihrer definierten Stumpfheit nicht in die Bowman-Membran ein, sondern drückt gegen die Bowman-Membran, ohne diese zu verletzen. Das Epithelium wird dadurch während der Schneidbewegung an der Bowman-Membran entlang geschoben. Dieser Vorgang ist relativ einfach und erfordert keine allzu große Geschicklichkeit.

Das Epithelium wird ähnlich wie bei der bisherigen LASIK-Methode nicht vollständig abgehobelt, sondern es wird ein Teil als "Scharnier" (hinge) belassen, um das das beiseite geschobene Gewebe geklappt wird. Anschließend wird das freiliegende Gewebe mit Hilfe eines Excimer-Lasers bearbeitet, um die Fehlsichtigkeit zu korrigieren. Danach wird das geschnittene und beiseite geschobene Epithelium wieder auf das bearbeitete Gewebe zurückgeschoben.

## Patentansprüche

1. Messer (3) für ein Mikrokeratom mit einer Klinge mit einem Klingenradius (R) und einem tangentialen Übergang zwischen einer Rundung des Klingenradius (R) und einer Freifläche (12, 13) der Klinge **gekennzeichnet durch** einen Schneidenwinkel (β) in einem Bereich von mehr als 30° bis max. 35°, einen Freiwinkel (α) in einem Bereich von 0° bis 4°, und einen Klingenradius (R) in einem Bereich von 150nm bis 800nm.

2. Messer nach Anspruch 1, wobei der Klingenradius vorzugsweise in einem Bereich von 200nm bis 600nm liegt.

3. Messer nach dem vorhergehenden Anspruch, wobei der Klingenradius weiter vorzugsweise in einem Bereich von 250nm bis 500nm liegt.

4. Verwendung eines Messers nach einem der vorhergehenden Ansprüche in einem Mikrokeratom.

5. Mikrokeratom (1) mit einem Schneidkopf (2), einer Applanationsfläche (4) und einem Messer (3) nach einem der vorhergehenden Ansprüche 1 bis 3.

6. Verwendung eines Mikrokeratoms (1) nach dem vorhergehenden Anspruch nach der LASEK-Methode.

## Claims

1. Knife (3) for a microkeratome having a blade with a blade radius (R) and a tangential transition between a round profile of the blade radius (R) and a flank (12, 13) of the blade, **characterized by** a cutting-edge angle (β) in a range from more than 300° to a maximum of 35°, a clearance angle (α) in a range from 0° to 4° and blade radius (R) in a range from 150 nm to 800 nm.

2. Knife according to Claim 1, wherein the blade radius is preferably in a range from 200 nm to 600 nm.

3. Knife according to either of the preceding claims, wherein the blade radius is more preferably in a range from 250 nm to 500 nm.

4. Use of a knife according to any one of the preceding claims in a microkeratome.

5. Microkeratome (1) having a cutting head (2), an applanation surface (4) and a knife (3) according to any one of the preceding Claims 1 to 3.

6. Use of a microkeratome (1) according to the preceding claim according to the LASEK procedure.

## Revendications

1. Couteau (3) pour un microkératome, comprenant une lame avec un rayon de lame (R) et une jonction tangentielle entre une courbure du rayon de lame (R) et une face de dépouille (12, 13) de la lame, **caractérisé par** un angle de coupe (β) supérieur à 30° et inférieur ou égal à 35 ° max., un angle de dépouille (α) compris entre 0° et 4°, et un rayon de lame (R) compris entre 150 nm et 800 nm.

2. Couteau selon la revendication 1, le rayon de lame étant de préférence compris entre 200 nm et 600 nm.

3. Couteau selon la revendication précédente, le rayon de lame étant en outre de préférence compris entre 250 nm et 500 nm.

4. Utilisation dans un microkératome d'un couteau selon l'une des revendications précédentes.

5. Microkératome (1) avec une tête de coupe (2), une surface d'applanation (4), et un couteau (3) selon l'une des revendications 1 à 3.

6. Utilisation suivant la méthode de LASEK d'un microkératome (1) selon la revendication précédente.
